# EUROPEAN PATENT APPLICATION

(11) **EP 4 595 911 A1**
(43) Date of publication of application: **06.08.2025**
(21) Application number: 24212500.3
(22) Date of filing: 12.11.2024
(51) Int. Cl.: A61B 18/12, A61B 18/14, A61B 34/20

(54) **SYSTEMS AND METHODS OF PULSED FIELD ABLATION**

(30) Priority: 05.02.2024 US 202418432142
(71) Applicant: Biosense Webster (Israel) Ltd., 2066717 Yokneam (IL)
(72) Inventor: GOVARI, Assaf, 2066717 Yokneam (IL); FUCHS, Amit, 2066717 Yokneam (IL); ALTMANN, Andres Claudio, 2066717 Yokneam (IL)
(74) Representative: Carpmaels & Ransford LLP

(57) **Abstract**

Systems and methods to process motion signals obtained from at least one motion sensor during delivery of plurality of PFA pulses or pacing signals to heart tissue location(s) and determine degrees of diaphragm spasm affected by the PFA or pacing signals. In some embodiments the system identifies reduction of diaphragm spasm during delivery of PFA pulses to a certain location and upon such identification indicates that continued delivery of further PFA pulses to that certain location may cause irreversible damage to the Phrenic nerve. Additionally, or alternatively, the system utilizes the diaphragm spasm degrees measured following pacing or ablation of at least two heart tissue locations, to map the degrees of spasm affected by the ablation of various heart tissue locations and thereby enables to conduct ablation at location associated with reduced spasm.

## Description

### TECHNOLOGICAL FIELD

The present invention relates to medical systems, in particular, but not exclusively, to cardiac ablation using irreversible electroporation (IRE) via pulsed field ablation.

### BACKGROUND

Diagnosis and treatment of cardiac arrhythmias include mapping the electrical properties of heart tissue, especially the endocardium, and selectively ablating cardiac tissue by application of energy. Such ablation can cease or modify the propagation of unwanted electrical signals from one portion of the heart to another.

A typical ablation procedure involves the insertion of an ablation catheter having a one or more electrodes at its distal end into a heart chamber, such that at least one of the electrodes is in electrical contact with a site of aberrant electrical activity therein, and operating that electrode with electrical signals affecting ablation of the sited having the aberrant electrical activity.

One ablation technique more recently in practice is Pulse Field Ablation (PFA), in which Irreversible electroporation (IRE) is applied via short electrical pulses referred to in the following electrical PFA pulses, that generate high enough electrical fields (typically greater than 450 Volts per centimeter) to irreversibly damage the cells. In bipolar PFA ablation the electric field is generated between two ablation electrodes at the distal end of the catheter, thus forming local electric field/pulses at the target tissue. In unipolar PFA ablation, the electric field is generated between at least one ablation electrode at the distal end of the catheter and a return electrode that is typically placed on the subject's skin and has a relatively large surface area so as not to affect ablation of tissues near it.

PFA may be associated with certain adverse effects particularly relating to nerve stimulation which may occur during delivery of the PFA electrical pulses/or pacing signals to certain tissue regions during the treatment. Such nerve stimulation, if occurs, may be expressed-by/lead-to contraction/spasm of muscles associated with the stimulated nerves resulting with pain/discomfort to the treated subject, and in some cases may also cause nerve damage. Accordingly, there are some known in the art technique aimed at mitigating/reducing nerve stimulation or some of its adverse effects during PFA treatments.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** is a schematic illustration of an ablation system **10** for pulse field tissue ablation with an ablation control system adapted to monitor of spasm or other adverse effects of nerve stimulation which may be caused by the ablation in accordance with some embodiments of the present invention;
**Fig. 2** is a block diagram exemplifying a configuration of an ablation control system **100** according to an embodiment of the present invention;
**Figs. 3A** and **3B****,** are graphical illustrations of respectively a spasm map **300** and guiding indication **303,** which may be presented by system **100** to guide physician for ablation of tissue locations associated with reduced spasm;
**Fig. 4** is a flow chart exemplifying a method **400** for spatial mapping of spasm which may be implemented by the ablation control system **100** according to some embodiments of the present invention; and
**Fig. 5** is a flow chart exemplifying a method **600** for monitoring a temporal evolution of spasm during PFA ablation to thereby identify a risk of causing of nerve damage by continued ablation of a certain tissue location.

Like reference numerals are used in the figures to designate similar modules/elements of the present invention or elements/modules having like functionalities. Accordingly, unless otherwise specified, the description of modules/elements with reference to a certain embodiment of the invention should be understood to apply to all embodiments of the present invention, in which such module/element is incorporated.

### DETAILED DESCRIPTION OF THE INVENTION

In both types of bipolar and unipolar PFA ablation treatments, an electric field is generated/delivered through the subject's body between the electrodes that are connected to ablation energy generator, may affect stimulation of nerves residing in the pathway of ablation energy flow through the subject's body. The nerve stimulation may in turn cause involuntary contraction of a muscles or group of muscles associated with the stimulated nerves, during the PFA ablation treatment. Hereinafter the such involuntary contraction of a muscles due to the nerve stimulation is referred to as *spasm.* Spasm may be accompanied by bursts of pain and discomfort to the subject, and in some cases may provide indication of a risk for causing permanent damage to the stimulated nerves. For instance, when applying PFA ablation to a subject heart, the phrenic nerve may be inadvertently stimulated, which may be apparent by spasm of the diaphragm, and in severe cases may lead to damage of the phrenic nerve.

To this end, avoidance, or reduction, of spasm during PFA treatments is desired in order to reduce subject's discomfort during the treatment and also to avoid/reduce the risk for causing nerve damage. In cases where muscle spasm that is caused by stimulation of nerves cannot be completely avoided, it is still particularly important to avoid causing irreversible damage to the stimulated nerves. Specifically, it is important to avoid causing irreversible damage to the phrenic nerve, which controls the diaphragm and therefore the subject's respiration.

The technique of the present invention is designated to achieve these goals, and reduce or avoid spasm in PFA treatment (e.g. in bipolar or unipolar treatments), and/or at least reduce the potential for causing irreversible damage to nerves affected by the PFA ablation of the treated region.

In an aspect of the invention, the degrees of spasm affected by PFA ablation of several tissue locations in a target region of interest of the heart that is to be ablated, are inspected, either in a preliminary spasm inspection procedure/stage which may be conducted prior to the actual ablation treatment, and/or in real-time during the ablation treatment. The degrees of spasm, which are measured in response to ablation or pacing of the several (two or more) inspected locations, are used to produce a spatial spasm mapping that spatially associates tissue locations in the region of interest to be ablated, with the degrees of spasm expected by PFA ablation of these locations (the term pacing here designate delivery of electric signal not affecting ablation or causing only a reversible electroporation). The spatial spasm mapping may then be used to display/provide physician with a spatial map showing the degrees of spasm expected by ablation of the different locations in the tissue region of interest (e.g. to enable a physician to a-priory plane a course/path for PFA ablation having reduced spasm prior to the actual PFA treatment); and/or to produce guidance indicia during the PFA treatment, to guide the physician to avoid ablation of tissue location associated with excessive nerve stimulation or spasm. For instance, during PFA ablation, gradient(s)/difference(s) between the degree of spasm affected by ablating the location, at which the ablation electrode at the distal tip of the catheter resides, and the degree(s) of spasm other location(s) in the tissue regions of interest, may be calculated based on the spatial spasm mapping, and further used to provide guidance indication to the physician about directions/location to which the ablation electrode/catheter should be moved in order to avoid or reduce the affected spasm.

In another aspect, the invention provides a technique to avoid and/or reduce the risk of causing irreversible damage to the nerves (e.g., to the phrenic nerve) during the ablation treatment. This aspect of the invention is based on the real-time monitoring, during the PFA treatment, of certain symptoms indicative of excessive nerve stimulation which may cause the irreversible damage. In particular, one such symptom is a reduction of the degree of spasm affected during PFA ablation of a certain tissue location. Indeed, in case spasm is affected by PFA ablation of a certain tissue location, the spasm may be expected to persist (e.g., with substantially similar degree) as long as the PFA pulses are continued to be delivered to substantially the same tissue location. However, in case nerve damage is starting to develop (even before it becomes irreversible) in the stimulated nerve affecting the spasm (which may be non-permeate damage at this stage), the degree of spasm may reduce (e.g., due to reduced response of the nerve to the stimulation caused by the PFA pulses). Therefore, the inventors of the present invention have realized that by monitoring the degree of spasm affected in real-time during PFA ablation of a certain tissue location and searching for reduction in the affected degree of spasm, it is possible to identify whether continued ablation of that tissue location might cause nerve damage. Therefore, according to this aspect of the invention, the degree of spasm is monitored/measured during PFA ablation of each certain tissue location, and upon identification of reduction/diminished spasm degree that not associated with a change in the ablation electrode's location, an indication may be issued to inform the physician that further continued PFA ablation of that certain location may affect irreversible nerve damage, or the PFA treatment/pulses may be automatically halted or modified (e.g. by reduction of the PFA pulses' intensity or repetition rate) in order to prevent the nerve damage.

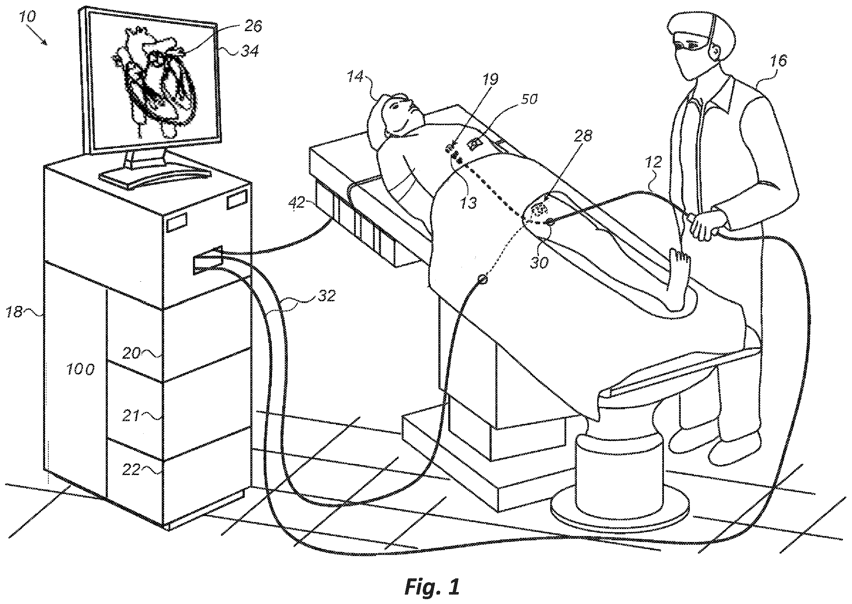

Reference is initially made to **FIG. 1****,** which is a schematic illustration of a system **10** for ablating tissue of a subject **14** while avoiding/reducing spasm or other effects of nerve stimulation, in accordance with some embodiments of the present invention. More specifically system **10** is configured and operable for carrying out *Pulsed Field Ablation* (PFA) also known as IRE ablation, in bipolar and/or unipolar modes.

System **10** includes an ablation catheter **12,** having a distal tip **13** with one or more ablation electrode(s) **19** thereon adapted to contact and ablate a target tissue location whose ablation is thought. Catheter **12** is inserted, by a physician **16,** into subject **14.** For example, catheter **12** may be inserted, via an insertion point **30,** into vasculature of the subject, and the distal tip **13** thereof may then be navigated to a particular location within the subject's body (e.g., within the heart at which a target tissue to be ablated is located).

Typically, catheter **12** includes, at the distal end **13** thereof, a position sensor (not specifically shown in the figure) that provides data/signals indicative of the real-time position of the distal end **13** of the catheter (the term *position* herein should be understood as refereeing to a location and/or orientation relative to the subject's **14** body). Accordingly, the position of the of the ablation electrode **19** within (relative to) the subject's body/target-tissue, is trackable by the system **10** thus enabling the physician **16** to place the ablation electrode **19** at specific target tissue whose ablation is desired and apply the PFA at said location. It should be understood that in various embodiments the system may include/or be connectable to additional position sensors, which may be for instance arranged in other medical devices, and may be adapted to track the positions of these sensors as well.

In some embodiments the position sensor(s) are magnetic position sensors operation in conjunction with location pad **42** which includes a plurality of location signal transmitters (e.g., magnetic coils) that generate/transmit electro-magnetic location signals (e.g., magnetic fields) in a predefined working volume surrounding the patient. Real time position of the distal tip **13** of the catheter **12** may then be tracked relative to the patient's body based on the magnetic/electro-magnetic location signals that are generated with location pad **42** and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, which are incorporated herein by reference.

According to embodiments of the present invention the system 10 is adapted to perform PFA ablation, by delivering PFA pulses/signals through at least one ablation electrode **19** on the catheter's **12** distal end **13.** The system may be adapted to perform PFA ablation in a bipolar mode and/or in unipolar mode, whereby in the former case the PFA pulses/signals are delivered between at least two ablation electrodes **19** of the catheter **12** and in the latter the PFA pulses/signals are delivered between at least one ablation electrode **19** of the catheter **12** and one or return electrode patch(s) e.g. **28,** which may optionally be coupled to the subject **14** (e.g. placed on the subject's **14** skin or other body tissue).

Generally, according to embodiments of the present invention, the system **10** includes or is associated with one or more motion sensors **50** that may be arranged to sense motion of the patient's tissue/skin at certain regions of interest (ROIs). For example, motion sensors **50** may be arranged near certain muscles (e.g., the diaphragm) associated with respective nerves (e.g. the phrenic nerve).

During or following the delivery of pacing signals or PFA pulses of an ablation treatment, the system **10** is adapted to monitor the motion of the muscles, based on the signals obtained from the motion sensor(s) **50** associated therewith, and thereby determine whether spasm of muscle(s), such as the diaphragm muscle, is/are affected due to stimulation of respective nerve(s).

The system **10** includes a console **18** including one or more units that facilitate performance of the techniques described herein. Typically, system **10**/console **18** includes one or more processors **20** with memory or storage with appropriate operating software stored therein, and user interface capability, by which functions of the system **10** and/or its sub-systems **100, 21** and **22** described below, are implemented. The ablation electrode(s) **19** of the catheter **12** as well as optionally, return electrode patches **28** (in case of unipolar ablation treatment is performed) are typically connected to the console **18** of the system, via cables **32** and electrical interface(s) (such as a ports or sockets). The sensor(s) **50** are typically connected, wirelessly or by wires to the console **18** for providing data indicative of the motion sensed thereby, to the console **18.** Additionally position sensor(s) typically located on the distal tip **13** of the catheter **12** (not specifically shown) may also be connected to the console **18** (e.g., by cables **32)** to provide thereto position data/signals indicative of the real-time position of the distal end **13** of the catheter based on magnetic based position sensing described herein above, and particularly of the ablation electrode(s) **19** thereof.

Typically, the system **10,** e.g., console **18** thereof, further includes a user interface (UI) **34** typically including a display and user input devices (e.g., joystick, mouse, keyboard and/or other device(s)), adapted to facilitate performance of the ablation procedure by displaying relevant information to physician **16** and receiving therefrom respective instructions/inputs for implementing the ablation procedure.

Console **18** may provide multiple functions, optionally including (1) modeling the endocardial anatomy in three-dimensions (3D) and rendering the model or anatomical map for display via UI **34,** (2) displaying via UI **34** activation sequences (or other data) compiled from recorded electrograms in representative visual indicia or imagery superimposed on the rendered anatomical map, (3) displaying real-time location and orientation of multiple catheters within the heart chamber, and (4) displaying sites of interest such as places where ablation energy has been applied. One commercial product embodying elements of the system 10 is available as the CARTO^{™} 3 System, available from Biosense Webster, Inc., 31A Technology Drive, Irvine, CA 92618.

Console **18** also includes an PFA energy generator **22,** configured to generate the electric PFA pulses, that are to be delivered during PFA ablation through the ablation electrode(s) **19** of the catheter **12** to the target tissue, which is to be ablated. Optionally, the PFA signal generator **22** is also adapted to generate pacing currents/signals that can be directed to pass in similar manner as the electric PFA pulses, but without affecting ablation. Additionally, console **18** typically also includes/implements position tracking system **21** that processes the position data/signals obtained from position sensor(s) that may be furnished on medical devices, such as catheter **12,** which are connected to the system **10,** to determine their respective positions relative-to/within the patient's body. The position tracking system **21** is connected directly or indirectly for receiving position indicative signals from position sensor(s) at the distal tip **13** of the catheter **12,** and based thereon may determine the position of the ablation electrode(s) **19** relative to the subject's body/anatomy. According to the present invention, system **10** (e.g., console **18)** includes an ablation control system **100** that is connected to the ablation energy generator **22** and to the position tracking system **21** and adapted to monitor spasm occurring during PFA ablation treatment and/or during delivery of pacing signals prior to actual ablation. Ablation control system **100** is adapted to process signals/data received from the motion sensor(s) **50,** which may be furnished on the patient's body on one or more muscles whose spasm is to be monitored, to determine whether the motion sensed thereby in response to delivery of PFA/pacing signals through the ablation electrode(s) **19,** is indicative of muscle spasm caused by stimulation of nerve(s). In various embodiments of the invention the ablation control system **100** is adapted to monitor the occurrence of spasm in association with the location of the target tissue being ablated/paced (e.g., based on the position of the catheter's **12** distal end **13** tracked by the position tracking system **21).** Based on the monitoring, ablation control system **100** assess the development of the spasm as function of the ablation duration (time during which PFA pulses or pacing signals are delivered to a certain target tissue/location) and/or as function of the spatial location of the activated ablation electrode(s) **19.** Based on the development of spasm being assessed, the ablation control system **100** may issues indication/guidance to the physician indicative of whether ablation of the target tissue at that location may be continued or should preferably be halted (or the intensity/amplitude of the ablation pulses be reduced) to prevent/reduce nerve damage or spasm, and/or optionally issues guidance instructions indicative of alternative locations/directions to which the distal tip **13** of the ablation catheter **12** may be moved in order to reduce the spasm to thereby relieve effects of excessive nerve stimulation which may be caused by continued PFA ablation of the same tissue location. Alternatively, or additionally, in some embodiments the ablation control system **100** may automatically halt the ablation treatment (e.g., the delivery of PFA pulses), and/or modify their properties (e.g., intensity/repetition-rate), so as to reduce the nerve stimulation affected thereby.

For example, the ablation control system **100** may cause UI **34** to display the location/position of the catheter **12,** e.g., by superimposing an icon representing the distal tip **13** of the catheter, or the ablation electrode **19** thereof, over an image of the subject's anatomy (in this particular example the heart) at the specific location being ablated/paced and may further also present/provide indication of spasm and/or its degree, affected by the ablation/pacing of the tissue at which the distal tip **13** is located. In embodiments UI **34** may also present data indicative of the specific muscles, of those being monitored by sensor(s) **50,** to which spasm is affected. Alternatively, or additionally, in some embodiment the ablation control system **100** may cause the UI **34** to present a spatial spasm map showing the spasm degrees expected to be affected by ablation of different locations in the tissue region of interest (ROI) whose ablation is sought. The spasm map may be for instance overlayed over an anatomical image/map of the tissue ROI. The spasm map may be for instance presented prior to ablation treatment (e.g., following a preliminary spasm inspection stage described below), to enable physician **16** to plane the ablation treatment accordingly. Yet alternatively, or additionally, during ablation treatment, in case of spasm, or of spasm above a certain degree, the ablation control system **100** may cause the UI **34** to issue indication about direction(s) of nearby tissue locations, to which the catheter's distal tip may be moved to reduce the affected degree of spasm. The indication may be provided for example in the form of indicia displayed by display of UI **34,** such as directing arrow(s) presented in relation to the subject's anatomy, or in other forms, such as vocal indication/guidance. Yet alternatively or additionally, in some embodiments the ablation control system **100** monitors the development of the spasm during the time PFA pulses are delivered to certain region to identify characterizing features in the spasm time development that may be indicative of a risk for causing damage/irreversible damage to one or more nerves such as the phrenic nerves. The characterizing features may be for example a decline in the spasm intensity sensed/measured during administration of PFA pulses to the same tissue region. Upon such identification the ablation control system **100** may cause the UI **34** to display indicia, or otherwise provide a notification (e.g., by sound or other means) indicating that continued administration of PFA pulses to the same tissue location might affect nerve damage.

To this end, in some embodiments/implementations of the invention, one or more of the sensor(s) **50** may be arranged at/near respective muscles whose spasm is to be monitored (herein after referred to as the monitored muscles). The sensor(s) **50** typically include motion sensors which may be placed for instance on the patient's body (skin/tissue) near the monitored muscles that may experience spasm due to stimulation of nerves by the PFA treatment/pulses. The ablation control system **100** may be adapted to monitor the signals obtain from the sensor(s) **50** following delivery of a pacing signals or PFA pulses through the ablation electrode(s) **19** to identify/measure the occurrence of muscle spasm near the sensor(s) **50** in association with the position of the activated ablation electrode(s) **19** of the catheter **12** which deliver the pacing signals or PFA pulses (e.g. the position of the ablation electrode(s) **19** as obtainable from the position tracking system **21).** The ablation control system **100** processes the degree of the measured spasm as function of the space (location of the ablation electrode(s) **19)** and/or as function of the time duration PFA pulses are delivered, and thereby assesses the spatial distribution/map of spasm affecting locations in the tissue region of interest, and/or temporal development of spasm during the PFA treatment, and based therein issues guidance instructions or other indications, such as spatial spasm map, for guiding the physician to locations of reduced spasm/nerve stimulation, or providing indication/alerts for continued/halting of the ablation at certain one or more locations.

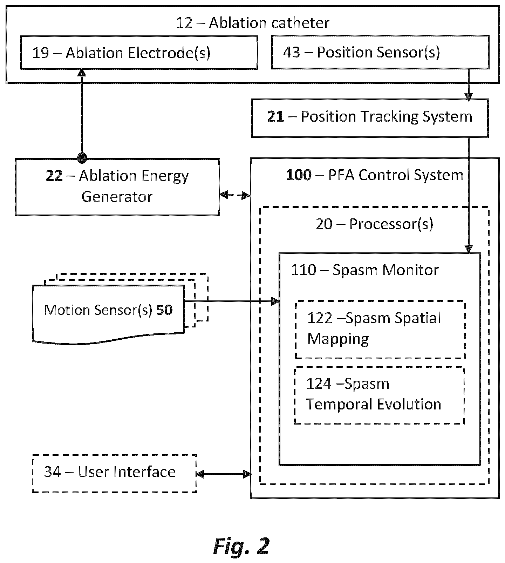

Reference is now made to **Figs. 2****,** which is a block diagram schematically illustrating in more detail the ablation control system **100** for controlling Pulse-Field Ablation (PFA) according to some embodiments of the present invention. The system **100** is adapted to monitor spasm during or prior to PFA ablation of a certain tissue region, to assess/indicate a risk for affecting irreversible nerve damage, for example to the phrenic nerve, based on characteristics of the identified spasm. The technique facilitates alerting/indicating the physician and/or automatic halting (or automatic reduction in PFA pulse intensities/amplitudes and/or reducing a rate/number of pulses of a PFA pulse train delivered for ablation of the certain tissue region), upon assessment that continued PFA ablation of the certain tissue region might cause the irreversible nerve damage.

As illustrated the ablation control system **100** is connected or connectable to an ablation catheter **12** having at least one ablation electrode **19** at a distal end **13** thereof. In some embodiments, the system **100** is capable/adapted for operating in bipolar PFA mode, and is connected or connectable to at least two ablation electrode **19** for delivery of PFA pulses between them. Alternatively, or additionally, in some embodiments, the ablation control system **100** is capable/adapted for operating in unipolar PFA mode and may also be connected or connectable to at least one (optional) return electrode patch **28** having a return electrode suitable for use in unipolar PFA ablation, such that PFA pulses can be delivered between the ablation electrode **19** and the electrode patch **28** in the unipolar PFA ablation.

As illustrated in **Fig. 2****,** the system is connected or connectable to one or more motion sensor(s) **50,** which are arranged or to be arranged at the subject's body near muscles whose spasm is to be monitored during the PFA treatment. For example, motion sensors **50** may include motion sensor arranged near the patient's **14** diaphragm for sensing spasm thereof, and/or optionally two motion sensor arranged/coupled respectively near the left and right side of the diaphragm, as well as optionally additional sensor(s) **50** for monitoring spasm of additional muscles.

The ablation control system **100** includes a spasm monitor **110,** which may be implemented for example via processor(s) **20** of system **10.** The spasm monitor **110** is connected directly or indirectly, wirelessly or by wired connection, to the one or more motion sensors **50** located/coupled to the subject's **14** body in vicinity of one or more muscles whose spasm are to be monitored. The spasm monitor **110** is adapted to monitor/process the motion signals obtained from the motion sensor(s) **50,** to identify whether the motion(s) sensed thereby manifests a spasm related motion pattern(s) indicative of muscle spasm of in one or more of the monitored muscles. Optionally in some implementations the spasm monitor **110** is also adapted to assess the degree/intensity-level of the sensed spasm and optionally a degree/level/magnitude of the sensed spasm based on the monitored motion signals.

Each motion sensor **50** may be implemented by, or include: a position sensor and/or an inertial measurement unit, and/or an accelerometer, and/or optionally also a gyro. The motion sensor(s) **50** may be adapted to provide the system **100,** wirelessly or by wired connection, signals indicative of changes in velocity or acceleration of muscles tissues coupled thereto from which spasm can be identified (e.g., based on inertial measurements performed by their IMUs/accelerometers). In some embodiments the motion sensors **50,** or any one or more of them, may also include, or be implemented by position sensors, trackable by the position tracking system **21.** In some implementations the motion sensor(s) **50,** or any one or more of them, may be registered to the patient's body (e.g., based on the registration of the location pads **42** such that the indication about identified thereby may be presented by UI **34** on top of an anatomical map illustrating the muscles or associated nerves (e.g., ROIs) being monitored by the respective sensor.

The spasm monitor **110** monitors the motion sensed by the motion sensors **50** for example by processing/filtering the signals obtained from each respective motion sensors **50** (which may be indicative of changes in position, velocity and/or acceleration of the monitored muscle coupled therewith), to identify spasm related motion pattern presented in the motion signals. Accordingly, the motion signals from motion sensor(s) **50** arranged near the diaphragm (e.g., near the right and/or left sides thereof), and/or near other muscles, may be processed in as described above to determine whether spasm occurs in the monitored muscles and/or its degree. Such processing may also be performed in some cases periodically or for multiple time frames to assess the development of spasm over time.

The spasm monitor **110** is also connected to the position tracking system **21** of system **10.** At least upon detection of spasm sensed by any of the sensors **50,** the spasm monitor **110** utilizes the position tracking system **21** to monitor/determine the location to which PFA pulses or pacing signals that are delivered cause the spasm in the patient's body (i.e. the position of the active ablation electrode(s) **19** at the catheter's distal tip **13** which delivers the PFA pulses/pacing signals). The spasm monitor **110** utilizes information about spasm occurring or not, and possibly its degree, in any of the monitored muscles **50** together with position data of activated ablation electrode(s) **19,** and based on that information determines/assess the affected spasm, or its degree, as function of the spatial location to which PFA pulses or pacing signals are delivered by the activated ablation electrode(s) **19,** and optionally also monitors the development of spasm in the monitored muscle(s) as function of time.

Based on this assessment, the PFA ablation control system **100** determines guidance/indications, and/or a spatial spasm map, to be provided to physician **16,** for reducing excessive stimulation/stimulation of nerves (at least those nerves that are associated with the muscles monitored by the motion sensors). Alternatively, or additionally, in some embodiments the PFA ablation control system **100** may be adapted or set to automatically interrupt the ablation operation (e.g., halt the PFA pulses or to automatically reduce their intensity/repetition-rate) to avoid nerve damage, and in particular to avoid irreversible nerve damage.

To this end, in various embodiments of the present invention the PFA ablation control system **100** may include/implement any one or both of the following optional spasm processing modalities, and based on any one or both of these modalities determine proper ablation indications (guidance/alerts) to be provided to physician **16,** and/or interrupt the normal operation of the ablation:
Processing Modality **122** - Spasm Spatial Mapping; and
Processing Modality **124** - Spasm Temporal Evolution.

### I. Optional Processing Modality 122 - Spasm Spatial Mapping:

Optionally, upon/during, or prior to, ablation of a certain target tissue region of interest, the modality **122** may be carried out in order to map the degrees of spasm that are, or would be, affected by ablation of several locations of the target tissue region (i.e., map the degrees of spasm as function of the location of the ablation electrode **19).** This thereby enables to identify preferred locations within the target tissue region at which application of the PFA ablation will yield reduced spasm and/or reduction in other adverse effects of excessive nerve stimulation.

To achieve that, with the Spasm Spatial Mapping modality **122** being operated prior to or during the actual ablation, the physician **16** may navigate the catheter's **12** distal end **13** to two or more locations within the target tissue region and deliver thereto pacing signals or PFA pulses via the ablation electrode(s) **19** of the catheter **12.** In turn, spasm monitor **110** inspects the spasm responses of the muscle(s) being monitored by the sensor(s) **50** to pacing signals or PFA ablation pulses delivered to the two or more locations. The spasm monitor **110** obtains from the position tracking system **21,** the respective two or more locations at which the ablation electrode(s) **19** delivers the pacing-signals or PFA-pulses and respectively determines the degrees/amplitudes of spasm affected by the delivery of the PFA pulses or pacing signals to these locations respectively. Spasm monitor **110** records/stores the respective degrees of spasm in association with the respective tissue locations to which the PFA pulses or pacing signals were delivered, and thereby maps the spasm degrees as function of the tissue location being paced/ablated.

As indicated above, the spasm spatial mapping modality **122** may be implemented during a preliminary spasm inspection stage, which may be carried out prior to actual ablation, and/or in real-time during the ablation procedure itself. In the former case, during the preliminary spasm inspection stage, physician **16** may inspect spasm response to ablation of the two or more tissue locations by delivering pacing signals thereto. During the delivery of the pacing signals to the two or more tissue locations, the spasm monitor **110** processes the signals sensed by the sensor(s) **50** to identify whether spasm related motion patterns are sensed thereby and the respective degrees/amplitudes of the sensed spasm related motions, and thereby maps the spasm responses as function of the locations to which the ablation electrode **19** delivers the pacing signals. In some implementations the spasm spatial mapping may be further extended to tissue locations not specifically inspected by the physician **16,** for example by interpolation or extrapolation of the spasm degrees sensed from the two or more inspected locations to additional tissue locations.

Alternatively, or additionally, the spasm spatial mapping modality **122** may be implemented in real-time during the ablation treatment. In this case, with each additional tissue location being ablated by delivery of PFA pulses thereto, the spasm monitor **110** processes the motion signals sensed by motion sensor(s) **50** and identifies whether spasm related motion patterns are sensed thereby and their respective degrees. The information about the spasm degrees affected by the ablation of different tissue locations may then be added/accumulated (e.g. in real time during the ablation treatment) to form or extend the spasm spatial mapping of spasm degrees as function of the ablated tissue locations (e.g. optionally forming the spasm spatial mapping on-the-fly during the treatment, and/or extending a spasm spatial mapping already being formed for instance in the preliminary inspection stage by adding thereto the information to about the spasm degrees affected by the ablation of the additional locations). Also, in this example the spatial mapping of the spasm degrees may be further extended to tissue locations not specifically inspected/ablated by the physician **16,** for example by interpolation or extrapolation of spasm degrees measured to additional tissue locations.

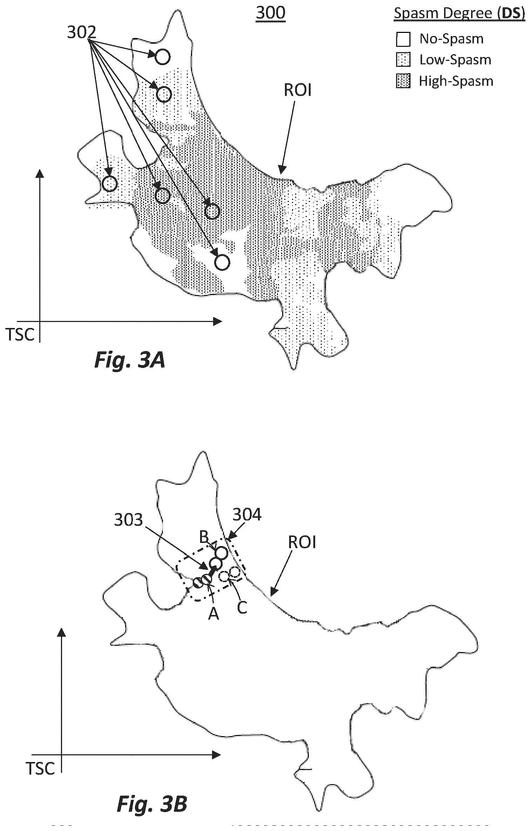

Optionally, based on the spasm spatial mapping **122,** a spasm map presenting spasm degrees as function of ablation location may be presented to physician **16** (e.g., via UI **34).** The spasm map may be presented to the physician during/after the preliminary spasm inspection stage, and/or during actual ablation, so as to enable physician **16** to set/decide about a course/path of tissue locations to be ablated, whose ablation will yield reduced spasm (or other adverse nerve stimulation effects). An example of such map **300** which may be presented to the physician **16** is schematically illustrated in **Fig. 3A** which is described in more details below.

Alternatively, or additionally, based on the spatial mapping of spasm degrees, the system **100** may provide physician **16** with guidance regarding the spasm degree expected by ablation of different tissue locations to allow him to navigate the catheter's **12** ablation electrodes to locations whose ablation are associated with reduced spasm. For example, in some implementations during the ablation of certain tissue location, system **100** may operate UI **34** to present imagery of the tissue region of interest **ROI** being ablated, with indicia that marks the location of the ablation electrode thereat, and with ablation guidance thereon (for example arrow(s) or other indicia, guiding the physician to move the catheter **12** (ablation electrode **19)** in directions of reduced spasm, and/or away from directions in which spasm is increased spasm. Such indicia may be determined for example based on gradients of the spasm degrees in the spasm spatial mapping, which is produced in real time during the treatment, or in the preliminary spasm inspection stage. The guiding indicia may be provided visually, for instance by guiding arrows presented on the UI **34** as indicated above, by vocal guidance or by other means. Alternatively, or additionally, in some embodiments during the ablation treatment, physician **16** may also operate the system **100** to display a map such as that illustrated in **Fig. 3A** showing the spasm degrees that would be affected by ablation/pacing of different locations in the tissue region of interest.

**Fig. 3A** exemplifies a spatial spasm map **300,** which may be presented to physician **16** based on the operation of the spasm spatial mapping modality **122,** according to embodiments of the present invention. A boundary of a certain tissue region of interest **ROI** to which ablation should be applied is marked in the figure. The locations **302** marked with small circles over the map **300,** designate several inspected tissue locations, the spasm responses to their respective ablation or pacing (e.g., reversible electroporation), were recorded/measured by the system **100.** In this non-limiting example, the map **300** is presented over tissue surface coordinates **TSC** whereby the degrees/levels of spasm are indicated by the different shades in the map **300.** In this none-limiting example three different shades are used to categorize the degrees of spasm affected by ablation of different tissue locations in three levels: no-spasm, low-spasm, and high-spasm. A map such as **300** presented in this figure may be used to present physician **16** with spasm degrees affected in one of the monitored muscles under ablation/pacing of different tissue locations **302,** or a computed collective degrees of spasm computed from the spasm degrees affected in a plurality of the monitored muscles in response to the ablation/pacing of the different tissue locations. In this non limiting example the map **300** is extended by interpolation and/or extrapolation to additionally also show the degrees of spasm that are expected to occur when ablating tissue locations other than the inspected points **302.** I.e., the degrees of spasm in the locations that were inspected by physician **16** are marked in the figure by the small circles over the map **M,** and the spasm degrees between these inspected points **302** are assessed by interpolation and/or extrapolation of the spasm degrees at the inspected points **302.**

In the non-limiting example of **Fig. 3B****,** a guiding indica **303** in the form of an arrow is illustrated. The guiding indicia may be presented over the tissue region of interest **ROI** which is being ablated. In the specific none limiting example shown in the figure, ablation around ostium **304** of a pulmonary vein is desired. The guiding indica **303** exemplifies guidance which may be provided to physician **16** in real-time during the ablation treatment around ostium **304,** to guide him to ablate locations of associated with lower spasm. In other examples, the ablation treatment may be an ablation line or area in other areas of a heart chamber, e.g., rooftop of a left atrium, area in the ventricles. For instance, the guiding indica **303** may be an arrow indicative of the gradient/difference in the degree of spasm affected between locations **A** and **B,** such that when physician **16** ablates tissue locations **A,** the system **100** guides him, e.g. in real time, that moving towards ablation of locations **B** will yield reduced spasm (for example as opposed to proceeding to ablation of tissue locations **C** which will also yield ablation across the desired area **304** but with higher degree of spasm).

Turning back to **Fig. 2****,** and particularly to the optional spasm spatial mapping modality **122,** in various embodiments spasm spatial mapping may be produced with as little as only two inspection locations **302** (or preferably more than two) at which spasm degree was inspected/measured. The spatial mapping may optionally be recorded/stored in a data form, such as an association/lookup-table, associating the different inspected locations **302** in the tissue region to be ablated with the respective spasm degrees measured in response to the ablation/pacing of these locations **302** respectively. Additionally, or alternatively, the spasm spatial mapping may include spasm degrees predicted for other locations in the tissue region of interest **ROI** e.g., in addition to those being inspected. The predicted spasm degrees may be obtained by interpolation and/or extrapolation of the spasm degrees measured from the inspected locations **302.**

Generally, a spasm spatial mapping as described above may be produced by the spasm monitor **110** per each motion sensor of the motion sensors **50** per each respective muscles of interest (i.e., per each muscle which is coupled with a respective motion sensor of sensors **50).** Accordingly, in some embodiments separate spasm spatial mappings may generally be separately produced by spasm monitor **110** separately per each of the monitored muscles, based on the processing of the motion signals obtained from the corresponding motion sensor thereof respectively. For instance, considering the system **100** is implemented with two motion sensors **50** respectively coupled to the left and right sides of the diaphragm. The spasm monitor **110** may utilize/process the motion signals from these respective sensors **50** to separately determine the spatial mapping of spasm degrees affected in the left and right sides of the diaphragm as function of the location of the ablating electrode **19.** The spasm spatial mappings produced per each motion sensor **50** in this example may include a 1^{st} mapping indicative the spasm affected on the left side of the diaphragm as function of the location of the ablation electrode **19,** and a 2^{nd} mapping indicative the spasm affected on the right side of the diaphragm as function of the location of the ablation electrode **19.** The spatial mapping of spasm of different muscles may generally be different as spasm of different muscles may be associated with stimulation of different nerves (in this example for instance the left diaphragm side may be more susceptible to stimulation of the left phrenic nerve and vice-versa right diaphragm side more susceptible to stimulation of the right phrenic nerve).

To this end, in certain embodiments/implementations, the spasm spatial mapping produced per each of the monitored muscles may be used independently by system **100** in order to present physician **16** with spatial maps, such as **300,** of spasm affected in each of the monitored muscles respectively, and/or to provide physician **16** with guidance/indicia **303** or alerts to avoid/reduce spasm in each of the monitored muscles.

Alternatively, or additionally, in some embodiments, the spasm monitor **110** is further adapted to combine the data from a plurality of spasm spatial mappings obtained respectively for a plurality monitored muscles, to form a collective spasm spatial mapping in which each tissue location **302** is associated with a certain collective degree of spasm, which is computed as function of the degrees of spasm that would be affected in several/all of the monitored muscles, according to the spasm spatial mappings of the individual muscles being monitored. Such a collective spasm spatial mapping may for example be produced by combining the spasm degrees sensed by the plurality of sensors **50** of the plurality of monitored muscles/nerves, as function of the location of the ablation electrode **19.** The use of a collective spasm mapping may be advantageous in certain scenarios in which a plurality (two or more) muscles are monitored, as it may simplify presentation of a spasm map **300** to physician **16** (e.g. enabling to present physician **16** with a single "collective" map **300** instead of one per each monitored muscle) and/or simplify the guidance and/or alerts given to the physician while possibly mitigating conflicting guidance instructions which may arise from separate spasm maps (for instance in case according to a 1^{st} spasm mapping of a 1^{st} muscle, the catheter should be moved in a 1^{st} direction in order to reduce spasm, while according to a 2^{nd} spasm mapping of a 2^{nd} muscle, the catheter should be moved in different 2^{nd} direction to reduce spasm of the 2^{nd} muscle).

To this end, according to certain embodiments of the present invention, the spasm monitor **110** may be adapted to produce a collective spasm spatial mapping, in which the spasm degrees from all the monitored muscles are incorporated to yield single collective spasm degree per each tissue location **302** of the ablation electrode **19** indicated in the collective mapping.

One approach, which may be implemented by system **100,** for producing the collective spasm spatial mapping based on the spasm spatial mappings of the individual muscles, is by aggregation (summation/integration) of the spasm degrees indicated in the spasm spatial mappings of the individual muscles, per tissue location, to determine thereby a collective spasm degree for each such tissue location. In some implementations of the invention, the collective spasm degree per each tissue location may be formed by mere summation/integration of the spasm degrees of the different muscles in that tissue location.

Alternatively, or additionally, in some embodiments the spasm monitor **110** may be adapted to compute the collective spasm degree per each tissue location by applying a differential summation/aggregation of the spasm degrees of the individual monitored muscles, in a manner giving higher weights to muscles experiencing higher degree of spasm for that respective tissue location, and lower weights to muscles experiencing lower spasm degrees in that location. In this regard the following should be noted:
(i) Different muscles may respond with different spasm amplitudes to the same level of stimulation applied to their associated nerves.
(ii) It is generally of interest during ablation to identify when a certain nerve is being particularly strongly stimulated and yields strong spasm in its associated muscle, while of less interest if several nerves are weakly stimulated yielding weak spasm of in each of their associated muscles (even in cases where the sum of the weak spasm amplitudes of the several weakly appears strong).

Therefore, in some embodiments of the present invention, the system **100,** may be configured and operable to generate the collective spasm mapping by differential summation/aggregation as indicated above in order to give higher weight to the muscles experiencing strong spasm and vice versa. To achieve that the spatial mapping modality **122** may be adapted to produce the collective spasm mapping by carrying out the one or more of the following:
(i) As for the 1^{st} issue (i) indicated above, in embodiments/implementations where the motion sensors are coupled to different muscles having different amplitudes of spasm responses to nerve stimulation, a normalization may be applied to the monitored spasm degrees that are obtained from the spasm monitor **110** for spasm in the different muscles. The normalization may be aimed to set the spasm degrees of the different muscles on a commune scale reflecting the spasm severity not only based on the amplitude of the spasm, but also to reflect the physiology of different muscles and the respective levels of nerve stimulations reflected by the spasm amplitudes in different muscles. Accordingly, in some implementations reference data indicative of relation between a degree/amplitude of spasm of each monitored muscle and the level of nerve stimulation affecting the respective agree of spasm in each of the muscles, may be used as physiological spasm normalization factors, by which the degrees of spasm in different ones of the monitored muscles may be normalized so as to place them on a common scale, before producing therefrom the collective spasm map.
(ii) As for the 2^{nd} issue (ii) indicated above, the spasm degrees (e.g., normalized as indicated in (i) above), per each inspection point **302,** may be aggregated in a manner giving higher weight to certain muscle's spasm which is associated with strong nerve stimulation, and lower weight weak muscles' spasm associated with weak stimulation nerves. For instance in certain implementations the system may apply a certain squashing function (e.g. sigmoid or any other suitable squashing function as will be appreciated by those versed in the art) to the degrees of spasm obtained from the spasm monitor per each monitored muscles (e.g. after they have being normalized normalized), in order to give higher weight to muscles experiencing higher spasm degrees/amplitudes in the commutative map, and then, per each inspected location **302** of the ablation electrode **19,** sum over the (e.g. normalized) squashed spasm degrees of the plurality of monitored muscles, to yield the map of commutative spasm degrees for the plurality of the monitored muscles per each inspected location **302,** such that it reflects the severity of spasm with higher weight to strongly stimulated muscles. Alternatively, in some implementations, the mapping of collective spasm degrees may be obtained by accounting for (e.g., summing over) only those muscles experiencing a high/maximal spasm degree (e.g., normalized), and for instance not accounting for (e.g., not including in the sum) the muscles whose degrees of spasm (e.g., normalized) is below a certain threshold. This may be done per each inspected location of the ablation electrode **19** to yield the commutative spasm map.

Thus, as indicated above, the system **100** may utilize the spasm spatial mapping(s) (e.g., a collective spasm spatial mapping, or muscle specific spasm spatial mappings) in order to present physician **16** with a map such as **300** illustrated in **Fig. 3A** illustrating the expected degrees of spasm to would be affected by ablation of different tissue locations. Typically, such a spasm map **300** may be presented to the physician **16** prior to the actual ablation treatment (e.g., after a preliminary spasm inspection stage by which spasm spatial mapping(s) may be constructed) so as to enable physician **16** to plan a course/path of ablation along the tissue region of interest while with reduced spasm effects. Alternatively, or additionally the spasm map **300** may also be presented to physician **16** at a later stage (e.g., according to his instructions), for example in real-time during the ablation treatment itself. Yet alternatively or additionally the spasm spatial mapping may be used by the system **100,** typically during the ablation treatment, in order to issue alerts and/or guidance instructions to the physician **16** so alert/guide him to conduct the PFA treatment while avoiding or reducing undesired/excessive nerve. To achieve that the spasm spatial mapping modality **122** utilizes the spasm spatial mapping(s) during the ablation treatment, in order to provide the physician **16** with indication/guidance (e.g., via graphical indicia **303** as in **Fig. 3B****,** or by other indicia e.g., audible) to navigate the ablation catheter to relatively "safe" location within the region of interest **ROI,** whose ablation is expected to yield reduced spasm. For instance, in some implementation based on a collective spasm spatial mapping and the current location of the ablation electrode **19,** an audible alert/guidance may be produced in order to guide the physician **16** to move the ablation electrode from its current location (e.g., in a certain direction) in order to avoid or reduce nerve stimulation.

Optionally, in embodiments/implementations in which spasm spatial map **300** such as that of **Fig 3A** or guiding indicia **303** such as that of **Fig. 3B** are graphically displayed, they may be displayed as an overlay over a model/image of the subject's anatomy at least partially including the **ROI** which is to be ablated. In such embodiments the system **100** may be capable of obtaining anatomy data (model or image) of the subject's endocardial anatomy (e.g., his heart or part thereof) in the **ROI** that is been treated by the PFA. As indicated above the endocardial anatomy may be modeled by system **10** based on imaging and/or based on tracking three-dimensional positioning of catheters inserted into the heart cavity.

The anatomy data (model or image) is generally registered with the subject's body, for instance based on location reference of the subject's body provided by the position tacking system **21,** and accordingly, the spasm spatial map **300** or the guiding indicia **303** (e.g. exemplified in **Fig 3A** and/or **Fig. 3B** respectively)are in some embodiments presented to the physician **16** as an overlay over the anatomical model or image with proper spatial registration. Optionally the system **100** may be further adapted to register the position of the catheter's distal tip **13** (e.g. or of an active ablation electrode(s) **19** thereof over the model/image of the anatomy, while optionally together with indicia indicating the degree of spasm affected at that position of the distal tip **13** and/or at surrounding locations (e.g. as obtained from the map **300),** and/or with indicia **303** indicating the expected gradient/difference of the spasm degree which will be affected upon movement of the catheter's distal tip **13** to one of the surrounding locations.

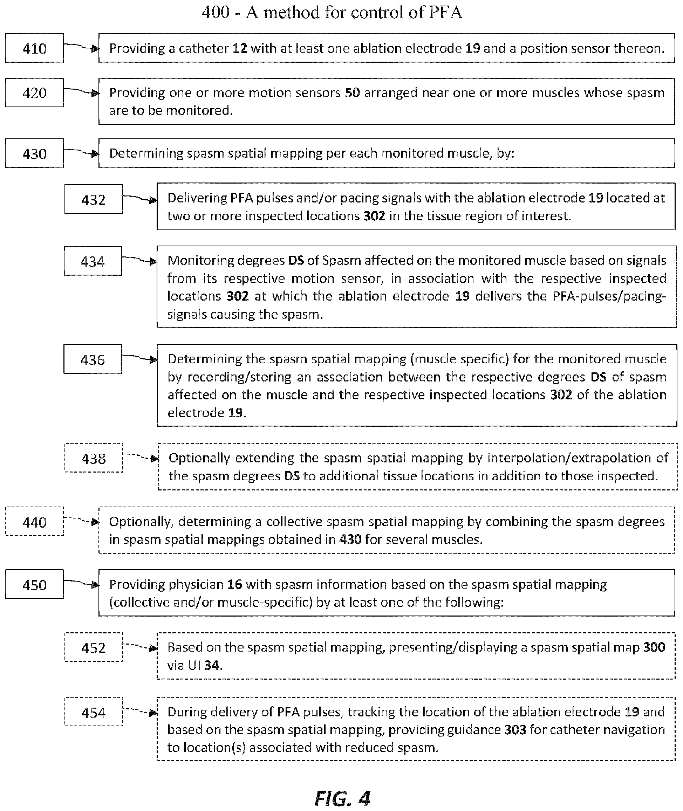

With reference to **Fig. 4****,** a flow diagram of a method **400** of the spasm spatial mapping modality **122** according to an embodiment of the invention is illustrated in self-explanatory manner. The method **400** may optionally be implemented by system **100** (e.g., via processor(s) **20** thereof) according to some embodiments of the present invention.

In operations **410** and **420,** an ablation catheter **12** having at least one ablation electrode **19** for PFA ablation is provided, as well as one or more motion sensors **50** which are to be arranged at various regions near muscles (such as the diaphragm) whose spasm are to be monitored during the PFA procedure.

In operation **430** spasm spatial mapping may be determined by carrying out of operations **432** to **438.** In operation **432** the system **100** enables physician **16** operate the catheter for delivering PFA pulses and/or pacing signals to the subject tissue while moving the ablation electrode to two or more spatial positions thereat. Operations **434** and **436** are carried out by system **100** during the delivery of the PFA pulses and/or pacing signals to monitor signals of a respective motion sensor(s) and thereby determine the degrees/levels of spasm affected on the muscle associated with that motion sensor (operation **434),** while also monitoring (operation **436)** two or more spatial locations **302** of the ablation electrode **19,** in conjunction with which the degrees/levels of spasm are determined in operation **434** (i.e. monitoring the ablation electrode's **19** position based on signals from the position sensor of the catheter which is tracked by the position tacking system **21).**

Operation **430** may be carried out to determine the spasm special mapping per each individual muscle that is being monitored by one of the sensors **50.** Operation **430** may be carried out in a preliminary spasm inspection stage, which may be carried out by physician **16** prior to the actual ablation, by delivery of pacing signals to two or more inspected locations **302** in the tissue region of interest, which is to be ablated, and/or in real-time during the ablation treatment during which PFA pulses are delivered to two or more inspected locations **302.** Operation **430** thus includes the following sub-operations:
- sub-operation **432,** in which pacing signals are delivered in the preliminary spasm inspection stage or PFA pulses are delivered in real-time, via the ablation electrode **19** located at two or more inspected locations **302** in the tissue region of interest.
- sub-operation **434** is carried out in parallel to sub-operation **432** and includes the monitoring degrees of spasm affected on the monitored muscle by processing the signals from its respective motion sensor **50** (e.g. to identify the amplitudes of spasm related motion pattern(s) presented thereby), in association with the respective inspected locations **302;**
- In sub-operation **436** the muscle specific spasm spatial mapping is determined for the monitored muscle, by recording/storing the association between the respective degrees of spasm affected on the muscle and the respective inspected locations **302** of the ablation electrode **19,** as determined in sub operation **434** for the two or more inspected locations **302;** and
- In optional sub-operation **438** the spasm spatial mapping for the specific muscle may be further extended by interpolation/extrapolation of the spasm degrees measured in **434** to assess the spasm degrees that would be affected by ablation of additional tissue locations in addition to those inspected in **432** and **434.**

It should be understood that operation **430** may be carried out in parallel (or sequentially/at-different-times) for the plurality of muscles whose spasms are being monitored by the sensors **50,** to thereby determine the muscle specific spasm spatial mappings for the plurality of muscles respectively (e.g., in case plurality of muscles are being monitored).

Optionally in some embodiments where plurality of muscles are being monitored, operation **440** may be carried out by the spasm monitor **110** to determine a collective spasm spatial mapping representing a collective spasm degree computed from the spasm degrees affected on the plurality of monitored muscles, per location of the ablation electrode **19.** This may be performed by combining the spasm degrees of the plurality of muscle specific spasm spatial mappings using any one of the techniques described above (e.g., in various embodiments normalization is applied to set the spasm degrees of different muscles on a common scale; and/or in various embodiments differential weighting is applied to assign, per inspected location, higher weights to muscles experiencing higher degree of spasm).

In operation **450** spasm information may be presented to physician **16** based on any one the muscle specific spasm spatial mapping(s) obtained in **430,** and/or optionally based on the collective spasm spatial mapping obtained in **440.** Operation **450** typically includes at least one of the following:
- Optional sub-operation **452** in which a spasm spatial map such as **300** in **Fig. 3A** may be displayed to physician **16** via UI **34.** The displayed map **300** may be based on any of the muscle specific spasm spatial mapping(s) obtained in **430,** or preferably in some embodiments based on the collective spasm spatial mapping obtained in **440** for the plurality of the monitored muscles. In some embodiments the spasm spatial map **300** may be displayed to physician **16** prior to the actual ablation treatment (e.g., after the optional preliminary spasm inspection stage) in order to allow physician **16** to plane a path/root of tissue ablation in the region of interest which will yield reduced spasm. Alternatively, or additionally, the spasm spatial map **300** may be displayed to physician **16** at any other stage (e.g., in real-time during the ablation treatment, and/or at any other time per request of the physician); and/or
- Optional sub-operation **454,** in which catheter navigation guidance (e.g., alerts and/or indica **303** as illustrated in **Fig. 3B****)** may be provided to physician **16** in real-time during the actual ablation treatment, so as to guide the physician to navigate the catheter **12** to locations having reduced spasm. Sub-operation **454** may include tracking the location of the ablation electrode **19** during the delivery of PFA pulses in the ablation treatment and based on the spasm spatial mapping (e.g., the muscle specific mappings obtained in **430,** or the collective mapping obtained in **440),** determining and providing physician **16** with guidance instructions/indicia to navigate the ablation electrode(s) **19** of catheter **12** to location(s) associated with reduced spasm. For example, in this operation the system **100** may utilize the (the spasm spatial mapping(s) to identify/determine one or more tissue location(s) in vicinity of the tracked location of the ablation electrode **19,** whose ablation is expected to yield reduced spasm and provide guidance/indication to the physician **16** to navigate the ablation electrode **19** towards these one or more tissue locations.

### II. Optional Processing Modality 124 - Spasm Temporal Evolution:

With reference back to **Fig. 2****,** upon/during ablation of each tissue location in the tissue region of interest **ROI,** the processing modality **124** may optionally be carried out by the system **100** to monitor the temporal development/evolution of spasm during the actual ablation of that tissue location and thereby assess/identify in case nerve damage might begin to develop by continued delivery of PFA pulses to that tissue location. To achieve that, the spasm monitor **110** tracks the location of the active ablation electrode(s) **19** and during the delivery of a train of PFA pulses to that target tissue location monitors changes in the spasm degree expressed by each certain muscle that is being monitored by sensor **50** to identify whether the changes in the spasm degree during the PFA pulse train is indicative of possible development of nerve damage which might occur by continuing the delivery of the PFA pulse train to that location.

To achieve that the spasm monitor **110** utilizes information from the position tracking system **21** to track the location of the active ablation electrode(s) **19** during the delivery of the PFA pulse-train. In parallel, the spasm monitor **110** processes the motion signals sensed from each specific muscle of interest to which a sensor **50** is coupled, and based on this processing determines a development in the spasm degree experienced by the specific muscle over time during the delivery of the PFA pulse train. Spasm monitor **110** is adapted to identify cases where the change/development of degree of spasm is characterized by a decrease in the degree of spasm in at least one monitored muscles (e.g. the diaphragm), which cannot be attributed to a change in the location of the ablation electrode(s) **19.** For instance, spasm monitor **110** may be adapted to identify a decrease in muscle's spasm occurring during a time the ablation electrode(s) **19** is active for delivering PFA pulses to substantially the same tissue location and associate such a decrease in the spasm degree with possible development of nerve damage in the nerve associated with the muscle. To this end, upon identifying that a decrease/reduction in the degree of spasm is substantial (e.g., above a certain threshold) and cannot be attributed to a change in the location of the ablation electrode **19,** the spasm monitor **110** determines/alerts that continuing the delivery of the PFA pulse-train to the same tissue location (e.g. without reducing the intensity of the PFA pulses) may further cause damage to the nerve associated with the muscle expressing the spasm decrease (e.g. a reduction in spasm of the diaphragm during ablation of the same location may indicated initial development of damage to the phrenic nerve).

For example, in some embodiments where the spasm monitor **110** implements the temporal evolution processing modality **124,** it may be adapted to halt/modify the ablatio and/or alert about a risk of causing nerve damage in case the following condition is identified during the delivery of a PFA pulse train to substantially the same tissue location:
The spasm expressed in a certain muscle being monitored is developed up to a certain spasm degree, and then the spasm degree declines/decreased, while the delivery of the PFA pulse-train persists to substantially the same tissue location (e.g. an identified decline/reduction in the spasm degree of the muscle, which is not attributed to a change in the location of the ablation electrode(s) **19** or to a change/reduction in the intensity of the PFA pulses).

In this case, the reduction/decline in the spasm degree of the muscle may likely be attributed to a nerve damage beginning to develop in the nerve associated with the monitored muscle (e.g., the development of the nerve damage may be causing the nerve, which is being stimulated by the PFA pulses, to stop reacting properly to the stimulation thereby resulting with the reduction in muscle spasm).

Thus, in embodiments where system **100** implements the spasm temporal evolution processing modality **124,** the spasm monitor **110** may be adapted process the spasm degree in at least one muscle (e.g. the diaphragm) being monitored by respective sensor **50** to identify cases where the spasm development over time presents the condition indicated above. In case the spasm development in the monitored muscle expresses this condition (e.g., reduction in spasm without apparent reason such as change in the ablation electrode position or PFA pulse intensity), the spasm monitor **110** may determine that nerve damage may be beginning to develop in the nerve associated therewith (e.g., the phrenic nerve in case the muscle being monitored is the diaphragm). Optionally the spasm monitor **110** may also operate to identify other conditions which may be associated with possible development of nerve damage expressed by the spasm's time development, such as in case during the delivery of the PFA pulses the spasm of a certain muscle is developed/increased above a certain threshold. In case of identification conditions indicative of a risk for causing nerve damage,, the spasm monitor **110** may optionally operate the UI **34** to issue respective indication/alert to the physician **16** about the risk of causing nerve damage by further delivery of the PFA pulses to that location at which the ablation electrode **19** (while possibly also indicating the muscle expressing the identified condition). Alternatively, or additionally, in case of identification that such a condition is expressed, the spasm monitor **110** may optionally operate (e.g., operate the ablation energy generator **22)** to halt further delivery of the PFA pulses, or reduce their intensity, repetition-rate or other properties of the PFA pulse-train delivery so as to prevent nerve damage.

As indicated above, in some embodiments the system **100** may be connected-with/include more than one sensor **50** (e.g., more than one motion sensor **50),** arranged at a different location of the patient's **14** body for monitoring spasm in different muscles, or muscle sections. To this end, it should be understood that in embodiments/implementations where several sensors **50** are connected to the system **100,** the spasm temporal evolution processing modality **124** may be carried out independently/separately for individual muscle(s)/muscle-section(s) that are being monitored by respective sensor(s) **50,** to separately assess whether spasm's time development in the individual muscles indicates a risk of damage to the respective nerves associated therewith.

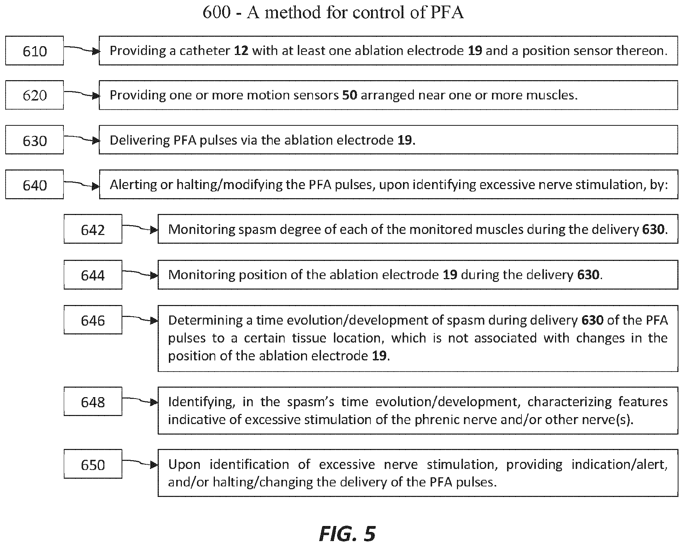

With reference to **Fig. 5****,** a flow diagram of a method **600** of the spasm temporal evolution processing modality **124** according to an embodiment of the present invention is illustrated in self-explanatory manner. The method **600** may optionally be implemented by system **100** (e.g., via processor(s) **20** thereof) according to some embodiments of the present invention.

In operations **610** and **620,** an ablation catheter **12** having at least one ablation electrode **19** for PFA ablation is provided, as well as one or more motion sensors **50** arranged near one or more muscles, such as near the left and right sides of the diaphragm, whose spasm temporal development is to be monitored during PFA treatment.

In operation **630** the system **100** facilitates operation of the catheter **12** by physician **16** for delivering PFA pulse-train to a tissue location in the region of interest of the subject **14** for ablating the tissue thereat. Operation **640** is carried out during the delivery of the PFA pulse-train in operation **630** to provide physician **16** with an alert, or halt/modify the delivery of the PFA pulse-train, in cases where excessive nerve stimulation affected by the delivery is identified based on the spasm temporal development. Operation **640** typically includes the following, which may be carried out separately for each of one or more of the muscles that are being monitored by corresponding ones of the sensors **50:**
Operation **642:** monitoring the spasm degree of the monitored muscle during the time duration of the PFA pulse-train delivery, based on the signals (e.g. motion signals) obtained from its respective sensor;
Operation **644:** monitoring the position of the ablation electrode **19** during the time duration of the PFA pulse-train delivery, based on the position signals obtained from position sensor of the catheter (e.g. as processed by the position tracking system **21);**
Operation **646** is then performed to determine/assess a time evolution/development of spasm in each monitored muscle during the delivery of the PFA pulse-train to a certain target tissue location. Optionally, operation **646** includes assessment of the part/portion in the development of the spasm degree of each monitored muscle, as obtained in **642,** which is not associated with changes in the location of the ablation electrode **19.** To this end, operation **646** may utilize/account-for the position of the ablation electrode **19** during the time of the PFA pulse delivery, to assess the development/change in spasm that is not attributed to changes in the position of the ablation electrode **19.** For instance, in some embodiments, operation **646** accounts for only those parts/portions of the spasm time development during which the ablation electrode remained substantially static/stationary at the same tissue location - thereby ignoring/filtering-out features of the spasm development which may be attributed to a change in the ablation electrode's **19** position. Alternatively or additionally, in some embodiments, particularly in embodiments, in which the spasm spatial mapping modality **122** is implemented by system **100,** the part/portion of the spasm time development that is not attributed to changes in the position of the ablation electrode **19,** may be assessed by normalizing (e.g. dividing) the spasm degree of the monitored muscle during the time duration of the PFA pulse as obtained in operation **642,** based-on/by the degrees of expected spasm at different tissue locations **302** at which the ablation electrode **19** was located during that time the spasm time development is monitored. To achieve that the degrees of expected spasm at different tissue locations **302** can be obtained based for example on those determined in operation **430** of the spasm spatial mapping modality **122** in method **400** described above. In a particular non limiting example such a normalization may be performed by mere division of the spasm degree sensed in operation **642** during respective time slots of the PFA pulse-train delivery, by the respectively expected degrees of spasm obtained from the spasm spatial dependency map of the spasm spatial mapping modality **122** for the specific position of the ablation electrode at the respective time slots. Accordingly, a normalized development of spasm in the monitored muscle during/as-function-of the time of PFA pulse-train delivery, which is not attributed to changes in the ablation electrode's location can be obtained in operation **646.** As will be appreciated by those versed in the art after knowing the invention, other techniques may be used instead of the normalization described above for accounting/compensating for changes in the ablation electrode's **19** position in the spasm temporal evolution.

Then, in operation **648** the spasm's time evolution/development, i.e. at least its portion/part that is not associated with changes in the ablation electrode's position, may be processed by the spasm monitor **110** to identify characterizing features indicative of excessive stimulation of the nerve associated with the monitored muscle whose spasm temporal development is assessed (e.g., the phrenic nerve in case of the diaphragm). One such characterizing feature, which is typically identified in operation **648,** is the condition indicated above, in which during the PFA ablation the spasm degree in a certain muscle reaches a certain level and then starts to decrease (without the decrease being attributed to halting/reduction of the PFA pulse-train or changing the delivery location of the PFA pulses). Identification of a decrease in the spasm degree during the ablation of a certain location, may indicate that the nerve associated with the certain muscle might starting to damage. Another characterizing feature of possible infliction of nerve damage, which may optionally also be assessed in operation **648,** is in case spasm degree of a certain muscle is increased above a certain level (maximal spasm degree threshold), without the increase being attributed to a change in the properties of the PFA pulses in the pulse train (e.g. their intensities/repetition-rate) or to a change in the tissue location pulses' delivery. Thus, upon recognizing these features, a determination may be made by the spasm monitor that the nerve associated with that certain muscle for which the modality **124** is applied, may be excessively stimulated by the PFA pulses, and may be damaged in case such excessive stimulation persists. Accordingly in these cases, in operation **650,** spasm monitor **110** may optionally operate the UI **34** to issue an alert/indication to physician **16** indicative of the excessive stimulation affected (possible indicating the nerve/muscle experiencing the excessive stimulation). Alternatively, or additionally, in operation **650,** spasm monitor **110** may optionally operate to automatically halt the PFA pulses or change their properties (e.g., reduce their intensities/frequency) to thereby reduce the nerve stimulation and prevent nerve damage.

As indicated above, the method **600,** may be carried out independently/separately for each muscle/muscle-section that is being monitored by one of the sensors **50** of the system **100,** in order to independently assess whether the nerve associated with the respective muscle experiences excessive stimulation, and in the latter case alert the physician or stop/alter the PFA treatment/pulses.

### Examples

Example 1. A method for Pulse-Field Ablation (PFA), the method includes:
providing a PFA system including an ablation catheter and a pulse-field ablation energy generator connectable to one or more ablation electrodes arranged on a distal tip of the catheter to facilitate tissue ablation near said distal tip via delivery of PFA pulses to at least one of said ablation electrodes;
providing at least one motion sensor to be arranged at at least one respective location on a patient's tissue/skin near the patient's diaphragm; and
the method includes processing motion signals obtained from the at least one motion sensor during a PFA treatment in which plurality of successive PFA pulses are delivered to a certain location of a heart tissue by said one or more electrodes, to determine degrees of spasm of said diaphragm caused by the plurality of successive PFA pulses delivered to said certain location, and identify reduction of spasm of the diaphragm affected by the delivery of the successive PFA pulses to the certain location; and upon identification that the reduction in the diaphragm's spasm exceeds a certain threshold, determining that continued delivery of further PFA pulses to the certain location may cause irreversible damage to the Phrenic nerve.

Example 2. The method according to example 1, wherein the at least one motion sensor includes at least two motion sensors to be arranged near the left and right sides of the diaphragm of a patient for sensing spasm of the diaphragm due to stimulation of left or right phrenic nerves by the PFA pulses or pacing signals.

Example 3. The method according to example 1 or 2, being adapted for use in bipolar PFA treatments.

Example 4. The method according to any one of examples 1 to 3, being adapted for use in unipolar PFA treatments.

Example 5. The method according to any one of examples 1 to 4, wherein the at least one motion sensor includes one or more of the following:
- one or more position sensors; and wherein the method includes processing the position signals from the one or more position sensors, and deriving the motion signals based on changes in the respective positions of the position sensors;
- one or more inertial measurement units (IMUs) adapted to provide said motion signals based on inertial measurements performed thereby; and
- one or more accelerometers adapted to provide the motion signals.

Example 6. The method according to any one of examples 1 to 5, wherein the at least one motion sensor is a wireless sensor including a wireless communication utility capable of wirelessly communicating the motion signals to the PFA system.

Example 7. A method for Pulse-Field Ablation (PFA), the method includes:
providing a PFA system including: an ablation catheter; a pulse-field ablation energy generator connectable to one or more ablation electrodes arranged on a distal tip of the catheter to facilitate tissue ablation near the distal tip via delivery of PFA pulses to at least one of the ablation electrodes; and a position tracking system that is connectable to a position sensor furnished near the distal tip of the catheter and adapted to track the position of the one or more ablation electrodes at the distal tip;
providing at least one motion sensor to be arranged near a patient's diaphragm;
processing motion signals obtained from the at least one motion sensor during delivery of a plurality of PFA pulses or pacing signals by the catheter to at least two respective locations of in a heart tissue of the patient, to determine spasm degrees associated with the delivery of the PFA pulses or pacing signals to the at least two heart tissue locations respectively; and associating the spasm degrees with the at least two heart tissue locations and thereby mapping degrees of diaphragm spasm affected by delivery of PFA pulses or pacing signals to two or more heart tissue locations.

Example 8. The method according to example 7, including utilizing said mapping in real time during PFA treatment in which PFA pulses are delivered to a certain location of the heart tissue, to determine at least one other tissue location in vicinity of the certain location that is associated, according to the mapping, with a reduced degree spasm relative to the degree of spasm affected by ablation of the certain location; and issuing a guidance indication for ablation of the other tissue location.

Example 9. The method according to example 7 or 8, including utilizing the mapping to display a map showing the degrees of spasm that are expected to be affected by ablation of various tissue locations in a region of interest of a heart tissue which is to be ablated.

Example 10. The method according to any one of examples 7 to 9, wherein the mapping is performed in at least one of the following:
- during a spasm inspection stage, which is carried out prior to PFA ablation treatment, and in which the plurality of pacing signals are delivered to the at least two respective locations of the heart tissue; and
- in real time during PFA treatment, in which said plurality of PFA pulses are delivered to said at least two respective locations of the heart tissue.

Example 11. The method according to any one of examples 1 to 10, wherein the at least one motion sensor includes at least two motion sensors to be arranged near the left and right sides of the diaphragm of a patient for sensing spasm of the diaphragm due to stimulation of left or right phrenic nerves by the PFA pulses or pacing signals.

Example 12. The method according to any one of examples 1 to 11 being adapted for use bipolar PFA treatments.

Example 13. The method according to any one of examples 1 to 12 being adapted for use unipolar PFA treatments.

Example 14. The method according to any one of examples 1 to 13, wherein the at least one motion sensor includes one or more of the following:
- one or more position sensors; and wherein the method includes processing position signals from the one or more position sensors and deriving said motion signals based on changes in the respective positions of the position sensors;
- one or more inertial measurement units (IMUs) adapted to provide the motion signals based on inertial measurements performed thereby; and
- one or more accelerometers adapted to provide the motion signals.

Example 15. The method according to any one of examples 1 to 14, wherein the at least one motion sensor is a wireless sensor including a wireless communication utility capable of wirelessly communicating the motion signals to the PFA system.

Example 16. A system for Pulse-Field Ablation (PFA), wherein the system is connectable to:
an ablation catheter having one or more ablation electrodes at a distal end thereof;
a position sensor arranged on ablation catheter providing data indicative of a position of the distal end of the ablation catheter; and
at least one motion sensor for coupling respectively with, or near, at least one region of a subject's body near the diaphragm;
a PFA energy generator connectable to the one or more ablation electrodes arranged on the distal end of the ablation catheter to facilitate tissue ablation near the distal end via delivery of PFA pulses to at least one of the ablation electrodes; and
wherein the system includes at least one processor adapted to carry out at least one of the following:
   **(a)** process motion signals obtained from the at least one motion sensor during a PFA treatment in which plurality of successive PFA pulses are delivered to a certain location of a heart tissue by said one or more electrodes, to determine degrees of spasm of the diaphragm caused by the plurality of successive PFA pulses delivered to the certain location, and identify reduction of spasm of the diaphragm affected by the delivery of the successive PFA pulses to the certain location; and upon identification that the reduction in the diaphragm's spasm exceeds a certain threshold, determining that continued delivery of further PFA pulses to the certain location may cause irreversible damage to the Phrenic nerve;
   **(b)** process motion signals obtained from the at least one motion sensor during delivery of a plurality of PFA pulses or pacing signals by the catheter to at least two respective locations of a heart tissue of the patient, to determine spasm degrees associated with the delivery of the PFA pulses or pacing signals to the at least two heart tissue locations respectively; and associating the spasm degrees with the at least two heart tissue locations, thereby mapping degrees of diaphragm spasm affected by delivery of PFA pulses or pacing signals to two or more heart tissue locations.

Example 17. The system according to example 16, wherein the at least one processor is adapted to utilize the mapping in real time during PFA treatment in which PFA pulses are delivered to a certain location of the heart tissue, to determine at least one other tissue location in vicinity of the certain location that is associated, according to the mapping, with a reduced degree spasm relative to the degree of spasm affected by ablation of the certain location; and issuing a guidance indication for ablation of the other tissue location.

Example 18. The system according to example 16 or 17, wherein the at least one processor is adapted to utilize the mapping to display a map showing the degrees of spasm that are expected to be affected by ablation of various tissue locations in a region of interest of a heart tissue which is to be ablated.

Example 19. The system according to any one of examples 16 to 18, wherein the at least one processor is adapted to perform the mapping in at least one of the following:
- during a spasm inspection stage, carried out prior to PFA ablation treatment, and in which the plurality of pacing signals are delivered to the at least two respective locations of the heart tissue; and
- in real time during PFA treatment, in which said plurality of PFA pulses are delivered to the at least two respective locations of the heart tissue.

Example 20. The system according to any one of examples 16 to 19, wherein the at least one motion sensor includes at least two motion sensors to be arranged near the left and right sides of the diaphragm of a patient for sensing spasm of said diaphragm due to stimulation of left or right phrenic nerves by the PFA pulses or pacing signals.

Example 21. The system according to any one of examples 16 to 20, wherein the at least one motion sensor comprises one or more of the following:
- one or more position sensors; and wherein the method comprises processing position signals from the one or more position sensors and deriving the motion signals based on changes in the respective positions of the position sensors;
- one or more inertial measurement units (IMUs) adapted to provide the motion signals based on inertial measurements performed thereby;
- one or more accelerometers adapted to provide the motion signals; and
- a wireless motion sensor including a wireless communication utility capable of wirelessly communicating the signals to the PFA system.

Example 22. The system according to any one of examples 16 to 21, wherein the at least one processor is adapted to process the motion signals to identify spasm related motion patterns therein, and determine the degrees of spasm based on amplitudes of the spasm related motion patterns in each of the motion signals.

Example 23. The system according to any one of examples 16 to 22 being adapted for use in at least one of a bipolar PFA treatment and a unipolar PFA treatment.

It should also be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof, which would occur to persons of ordinary skills in the art upon reading the description of the present invention and which are not disclosed in the prior art.

## Claims

1. A system for Pulse-Field Ablation (PFA),
the system is connectable to:
an ablation catheter having one or more ablation electrodes at a distal end thereof;
a position sensor arranged on ablation catheter providing data indicative of a position of the distal end of the ablation catheter; and
at least one motion sensor for coupling respectively with, or near, at least one region of a subject's body near the patient's diaphragm;
a PFA energy generator connectable to the one or more ablation electrodes arranged on the distal end of the ablation catheter to facilitate tissue ablation near said distal end via delivery of PFA pulses to at least one of the ablation electrodes; and
wherein the system comprises at least one processor adapted to carry out at least one of the following:
**(a)** process motion signals obtained from the at least one motion sensor during a PFA treatment in which plurality of successive PFA pulses are delivered to a certain location of a heart tissue by said one or more electrodes, to determine degrees of spasm of said diaphragm caused by the plurality of successive PFA pulses delivered to said certain location, and identify reduction of spasm of said diaphragm affected by the delivery of said successive PFA pulses to said certain location; and upon identification that said reduction in the diaphragm's spasm exceeds a certain threshold, determining that continued delivery of further PFA pulses to said certain location may cause irreversible damage to the Phrenic nerve;
**(b)** process motion signals obtained from the at least one motion sensor during delivery of a plurality of PFA pulses or pacing signals by said catheter to at least two respective locations of in a heart tissue of the patient, to determine spasm degrees associated with the delivery of the PFA pulses or pacing signals to the at least two heart tissue locations respectively; and associating said spasm degrees with said at least two heart tissue locations, thereby mapping degrees of diaphragm spasm affected by delivery of said PFA pulses or pacing signals to two or more heart tissue locations.

2. The system according to claim 1, wherein said at least one processor is adapted to utilize said mapping in real time during PFA treatment in which PFA pulses are delivered to a certain location of the heart tissue, to determine at least one other tissue location in vicinity of said certain location that is associated, according to said mapping, with a reduced degree spasm relative to the degree of spasm affected by ablation of said certain location; and issuing a guidance indication for ablation of said other tissue location.

3. The system according to claim 1 or claim 2, wherein said at least one processor is adapted to utilize said mapping to display a map showing the degrees of spasm that are expected to be affected by ablation of various tissue locations in a region of interest of a heart tissue which is to be ablated.

4. The system according to any preceding claim, wherein said at least one processor is adapted to perform said mapping in at least one of the following:
- during a spasm inspection stage, carried out prior to PFA ablation treatment, and in which said plurality of pacing signals are delivered to said at least two respective locations of said heart tissue; and
- in real time during PFA treatment in which said plurality of PFA pulses are delivered to said at least two respective locations of said heart tissue.

5. The system according to any preceding claim, wherein said at least one motion sensor comprises at least two motion sensors to be arranged near the left and right sides of the diaphragm of a patient for sensing spasm of said diaphragm due to stimulation of left or right phrenic nerves by the PFA pulses or pacing signals.

6. The system according to any preceding claim, wherein said at least one motion sensor comprises one or more of the following:
- one or more position sensors; and wherein the method comprises processing position signals from the one or more position sensors and deriving said motion signals based on changes in the respective positions of said position sensors;
- one or more inertial measurement units (IMUs) adapted to provide said motion signals based on inertial measurements performed thereby;
- one or more accelerometers adapted to provide said motion signals; and
- a wireless motion sensor comprising a wireless communication utility capable of wirelessly communicating said signals to the PFA system.

7. The system according to any preceding claim, wherein said at least one processor is adapted to process the motion signals to identify spasm related motion patterns therein, and determine said degrees of spasm based on amplitudes of said spasm related motion patterns in each of the motion signals.

8. The system according to any preceding claim adapted for use in at least one of a bipolar PFA treatment and a unipolar PFA treatment.
